# EUROPEAN PATENT SPECIFICATION

(11) **EP 4 129 662 B1**
(45) Date of publication and mention of the grant of the patent: **30.10.2024**
(21) Application number: 21781736.0
(22) Date of filing: 29.03.2021
(51) Int. Cl.: B32B 27/00, A61B 5/25, A61B 5/256

(54) **BIOSENSOR**
BIOSENSOR
BIOCAPTEUR

(30) Priority: 30.03.2020 JP 2020059650
(43) Date of publication of application: 08.02.2023
(73) Proprietor: Nitto Denko Corporation, Ibaraki-shi, Osaka 567-8680 (JP)
(72) Inventor: ODANE, Chiharu, Ibaraki-shi, Osaka 567-8680 (JP)
(74) Representative: Hoffmann Eitle
(86) International application number: PCT/JP2021/013247
(87) International publication number: WO 2021/200805

(56) References cited:
- JP-A- 2012 031 071
- JP-A- 2013 531 512
- JP-A- 2015 093 167
- JP-A- 2019 150 465
- JP-A- 2019 218 417
- US-A1- 2016 363 555
- US-A1- 2017 223 846

## Description

### [Technical Field]

The present invention relates to biological sensors.

### [Background Art]

Biological sensors for measuring biological information, such as electrocardiograms, pulses, electroencephalograms, or myoelectric waves, are used at medical institutions, such as hospitals or clinics, nursing homes, or homes. The biological sensor includes a biological electrode that is in contact with a living body and acquires a subject's biological information. When measuring the biological information, the biological sensor is affixed to a subject's skin to bring the biological electrode into contact with the subject's skin. The biological information is measured by acquiring an electrical signal related to the biological information with the biological electrode.

For the above-described biological sensor, a biocompatible polymer substrate is disclosed which includes, for example, a polymer layer having an electrode on one side, the polymer layer being formed by polymerizing dimethylvinyl-terminated dimethyl siloxane (DSDT) and tetramethyl tetravinyl cyclotetrasiloxane (TTC) with a predetermined ratio (see, for example, Patent Document 1).

In the biocompatible polymer substrate disclosed in Patent Document 1, the polymer layer is affixed to human skin, and the electrode detects a myocardial voltage signal from the human skin and receives and records the myocardial voltage signal in a data acquisition module.

### [Prior art documents]

### [Patent Documents]

[Patent Document 1] Japanese Unexamined Patent application publication No. 2012-10978

Further related art may be found in JP 2015093167A which describes a biological sensor and in US 20170223846A1 which describes a multi-part flexible encapsulation housing for electronic devices.

### [Summary of Invention]

### [Problem to be solved by the invention]

However, because the polymer layer of the biocompatible polymer substrate disclosed in Patent Document 1 is affixed to the living body, there is
a problem that the polymer layer tends to peel off from the skin due to movement of the skin, perspiration, or the like.
In particular, a biological sensor, such as a biocompatible polymer substrate, is often affixed to the skin for a long period of time, so that once the biological sensor peels away from the skin during use, biological information may not be stably measured.

According to one aspect of the present invention, it is an object to provide a biological sensor capable of being stably affixed to a living body.

### [Means for Solving Problems]

The present invention is defined by the appended independent claim. The dependent claims describe optional features and distinct embodiments.

According to an aspect of the present invention, a biological sensor that is to be affixed to a living body and is for acquiring a biological signal, includes
a cover member; and
a porous substrate having a porous structure, the porous substrate being disposed on the cover member on a side of the living body,
a sticking layer, including the porous substrate and a first adhesive layer that is disposed on the porous substrate on a side of the living body, exhibiting a shear stress of from 5×10⁴ N/m² to 65×10⁴ N/m² when the sticking layer is deformed in a direction perpendicular to a thickness direction of the sticking layer by 5% to 15% of a length of the sticking layer, and
a moisture permeability of the sticking layer being within a range from 65 g/m²·day to 4000 g/m²·day.

### [Effects of the Invention]

According to an aspect of the present invention, a biological sensor can be stably affixed to a living body.

### [Brief Description of Drawings]

[FIGURE 1] FIG. 1 is a perspective view illustrating a configuration of a biological sensor according to an embodiment of the present invention.
[FIGURE 2] FIG. 2 is an exploded perspective view of FIG. 1.
[FIGURE 3] FIG. 3 is a cross-sectional view of I-I in FIG. 1.
[FIGURE 4] FIG. 4 is a plan view illustrating a configuration of a sensor unit.
[FIGURE 5] FIG. 5 is an exploded perspective view of a part of the sensor unit shown in FIG. 3.
[FIGURE 6] FIG. 6 is an explanatory view illustrating a state in which the biological sensor is affixed to skin of a living body (an analyte).
[FIGURE 7] FIG. 7 is an explanatory diagram illustrating an example of a test method for measuring shear stress at 10% deformation of a sticking layer.
[FIGURE 8] FIG. 8 is an explanatory diagram illustrating an example of a test method for measuring shear stress at 30% deformation of the biological sensor.
[FIGURE 9] FIG. 9 is an explanatory diagram illustrating a peeling position of the biological sensor.

### [Mode for Carrying Out the Invention]

In the following, embodiments of the present disclosure will be described in detail. To facilitate understanding of the description, in each drawing, to the same elements, the same reference numeral will be assigned, and an explanation may be omitted. Moreover, a scale of each member in the drawings may be different from the actual scale, unless otherwise indicated.

### <Biological sensor>

A biological sensor according to the present embodiment will be described. The term "living body" includes a human body (human) and an animal, such as a cow, a horse, a pig, a chicken, a dog, and a cat. The biological sensor according to the present embodiment can be suitably used for a living body, especially a human body. In the present embodiment, as an example, a case of a patch-type biological sensor affixed to skin that is a part of a living body to measure biological information will be described.

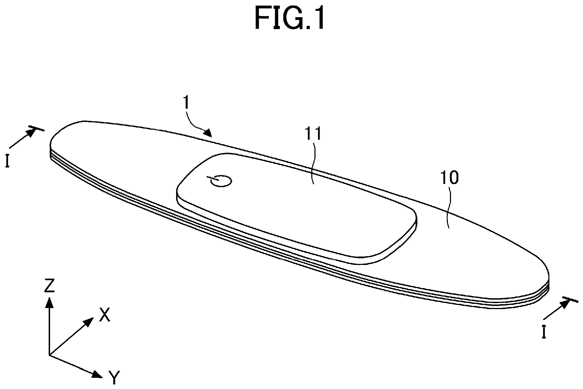

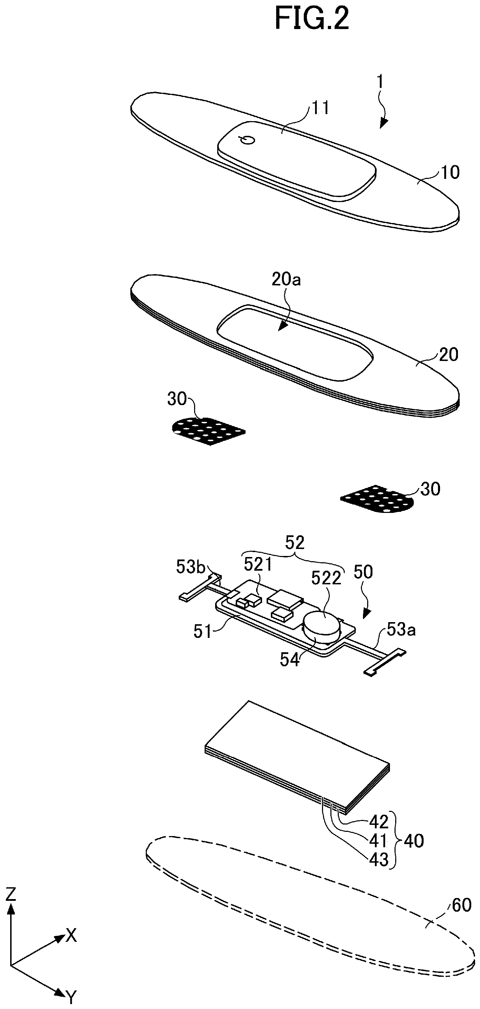

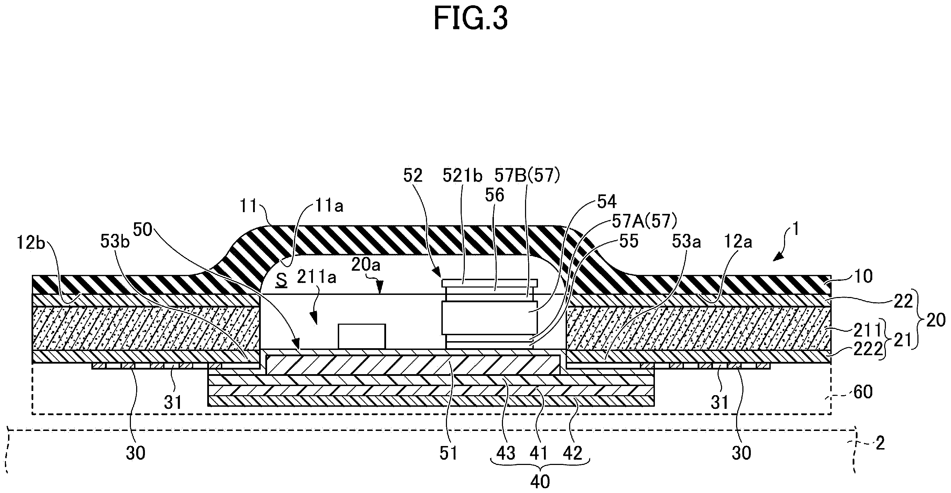

FIG. 1 is a perspective view illustrating a configuration of a biological sensor according to the present embodiment. FIG. 2 is an exploded perspective view of FIG. 1. FIG. 3 is a cross-sectional view of I-I in FIG. 1. As shown in FIG. 1, the biological sensor 1 is a plate-like (sheetlike) member that is approximately elliptically formed in a planar view. As shown in FIGS. 2 and 3, the biological sensor 1 includes a cover member 10, a first laminated sheet (first laminated body) 20, an electrode 30, a second laminated sheet (second laminated body) 40, and a sensor unit 50. The biological sensor 1 is formed by laminating the cover member 10, the first laminated sheet 20, the electrodes 30, and the second laminated sheet 40 from the cover member 10 side to the second laminated sheet 40 side in this order. The biological sensor 1 enables the first laminated sheet 20, the electrodes 30, and the second laminated sheet 40 to be affixed to skin 2 that is a living body to acquire a biological signal. The cover member 10, the first laminated sheet 20, and the second laminated sheet 40 have substantially the same external shape in a planar view. The sensor unit 50 is mounted on the second laminated sheet 40 and stored in a storage space S formed by the cover member 10 and the first laminated sheet 20.

In the specification of the present application, a three-dimensional orthogonal coordinate system in three axes (in an X-axis direction, a Y-axis direction, and a Z-axis direction) is used. A transverse direction of the biological sensor 1 is set to be the X-axis direction, the longitudinal direction of the biological sensor 1 is set to be the Y-axis direction, and the height direction (in the thickness direction) of the biological sensor 1 is set to be the Z-axis direction. A direction opposite to the side (sticking side) on which the biological sensor is affixed to the living body (analyte) is set to be a +Z-axis direction, and the side (sticking side) on which the biological sensor is affixed to the living body (analyte) is set to be a -Z-axis direction. In the following description, for convenience of illustration, the +Z-axis side will be referred to as an upper side or above, and the -Z-axis side will be referred to as a lower side or below. However, they do not represent a universal vertical relationship.

The biological sensor 1 exhibits a shear stress of from 5×10⁴ N/m² to 65×10⁴ N/m² when the sticking layer 21 that is a portion of the first laminated sheet 20 is deformed in a direction perpendicular to a thickness direction of the sticking layer 21 (X-axis direction and Y-axis direction) by 5% to 15% of a length of the sticking layer 21, and a moisture permeability of the sticking layer 21 is within a range from 65 g/m²·day to 4000 g/m²·day. The inventor of the present application has focused on reducing the shear stress when the sticking layer 21 is deformed in the longitudinal direction (in the X-axis direction and the Y-axis direction) to make the sticking layer 21 reasonably soft, and at the same time, increasing an air-permeability of the sticking layer 21, while making the moisture permeability of the sticking layer 21 be within a predetermined range, to make the biological sensor sufficiently flexible. The inventor found that according to the above-described configuration, even when the skin 2 is stretched due to contact pressure of the biological sensor 1 on the skin of the subject, movement of the living body (body movement), or the like, the stress at the interface between the first laminated sheet 20 and the second laminated sheet 40 and the skin 2 can be reduced, and thereby the biological sensor 1 can be prevented from peeling off from the skin 2.

The amount of deformation when the sticking layer 21 is deformed in a direction perpendicular to the thickness direction of the sticking layer 21 (in the X-axis direction and the Y-axis direction) is preferably 8% to 12% of the length of the sticking layer 21, more preferably 9.5% to 10.5%, and most preferably 10%.

The shear stress, when the sticking layer 21 is deformed in a direction perpendicular to the thickness direction of the sticking layer 21 (X-axis direction and Y-axis direction) by 5% to 15% of a length of the sticking layer 21, is preferably within a range from 5×10⁴ N/m² to 15×10⁴ N/m², and more preferably within a range from 6×10⁴ N/m² to 12×10⁴ N/m². In the case where the shear stress, when the sticking layer 21 is deformed by 5% to 15% of the length of the sticking layer 21, is within a range from 5×10⁴ N/m² to 15×10⁴ N/m², the flexibility of the sticking layer 21 can be further stably enhanced.

The moisture permeability of the sticking layer 21 is preferably within a range from 50 g/m²·day to 5000 g/m²·day, more preferably within a range from 2000 g/m²·day to 4800 g/m²·day, and further more preferably within a range from 2500 g/m²·day to 4500 g/m²·day. When the moisture permeability of the sticking layer 21 is within a range from 50 g/m²·day to 5000 g/m²·day, the flexibility of the first laminated sheet 20 can be more stably maintained.

The moisture permeability can be calculated using a publicly-known method, for example, a moisture permeability test called a cup method, a MOCON method, or the like. In the cup method, water vapor permeated through the material to be measured is absorbed by a hygroscopic agent in the cup, and moisture permeability is measured from a change in the weight of the absorbing agent. In the MOCON method, water vapor transmitted through the material to be measured is measured using an infrared sensor.

Moreover, the biological sensor 1 preferably exhibits the shear stress of from 5×10⁴ N/m² to 25N×10⁴ N/m² when 25% to 35% of the entire length of the biological sensor 1 (in the Y-axis direction) with respect to the contact surface with the skin 2 is deformed, more preferably from 5.6×10⁴ N/m² to 20×10⁴ N/m², and even more preferably from 6.9×10⁴ N/m² to 13×10⁴ N/m². When the shear stress is within the above-described ranges, the stress at an interface between the second laminated sheet 40 and the skin 2 can be reduced, so that the biological sensor 1 can be deformed more flexibly relative to the contact surface with the skin 2, and the biological sensor 1 can be prevented from peeling off from the skin 2.

An amount of deformation of the biological sensor 1 in the entire length direction (Y-axis direction) is preferably 28% to 32% of the length of the sticking layer 21, more preferably 29.5% to 30.5%, and most preferably 30%.

### [Cover member]

As shown in FIGS. 1 to 3, the cover member 10 is positioned outermost (in the +Z-axis direction) of the biological sensor 1 and affixed to the upper surface of the first laminated sheet 20. The cover member 10 has a projection portion 11 that protrudes with substantially a dome shape in the height direction (the +Z-axis direction) of FIG. 1 in the central portion in the longitudinal direction (the Y-axis direction). A concave portion 11a on the living body side is formed into a recessed shape inside (sticking side) the projection portion 11. The lower surface (on the sticking side) of the cover member 10 is formed flat. Inside the projection portion 11 (sticking side), a storage space S for storing the sensor unit 50 is formed by the concave portion 11a of the inner surface of the projection portion 11 and through hole 211a in a porous substrate 211.

The cover member 10 may be formed of a flexible material such as silicone rubber, fluorine rubber, urethane rubber, or the like. Moreover, the cover member 10 may be formed by laminating the above-described flexible material on a surface of a base resin, such as polyethylene terephthalate (PET), as a support. When the cover member 10 is formed using the above-described flexible material and the like, the sensor unit 50 disposed in the storage space S of the cover member 10 is protected, and an impact applied to the biological sensor 1 from the upper side is absorbed, thereby reducing an impact on the sensor unit 50.

Thicknesses of the upper surface and side walls of the projection portion 11 of the cover member 10 are greater than thicknesses of flat portions 12a and 12b disposed at both end sides of the cover member 10 in the longitudinal direction (Y-axis direction). Thus, flexibility of the projection portion 11 can be made lower than flexibility of the flat portions 12a and 12b, and thereby the sensor unit 50 can be protected from an external force applied to the biological sensor 1.

The thicknesses of the upper surface and the side walls of the projection portion 11 are preferably within a range from 1.5 mm to 3 mm, and the thicknesses of the flat portions 12a and 12b are preferably within a range from 0.5 mm to 1 mm.

Because the thinner flat portions 12a and 12b are more flexible than the projection portion 11, when the biological sensor 1 is affixed to the skin 2, the biological sensor 1 can be readily deformed conforming to deformation of a surface of the skin 2 caused by body movements such as stretching, bending, and twisting. Accordingly, a stress applied to the flat portions 12a and 12b when the surface of the skin 2 is deformed can be reduced, and thereby the biological sensor 1 can be made unlikely to peel off from the skin 2.

Outer peripheries of the flat portions 12a and 12b are shaped so that thicknesses gradually decrease toward the ends. Thus, the flexibilities of the outer peripheries of the flat portions 12a and 12b can be made further higher, and the wearing feeling when the biological sensor 1 is affixed to the skin 2 can be improved compared to a case where the thicknesses of the outer peripheries of the flat portions 12a and 12b are not reduced.

A hardness (strength) of the cover member 10 is preferably within a range from 40 to 70, and more preferably within a range from 50 to 60. When the hardness of the cover member 10 is within the above-described range, a third adhesive layer 42 provided on the sticking side (in the -Z-axis direction) of a second substrate 41 can readily reduce a stress at the interface with the skin 2 when the skin 2 is stretched by body movement. The hardness refers to Shore A hardness.

### [First laminated sheet]

As shown in FIG. 3, the first laminated sheet 20 is affixed to a lower surface of the cover member 10. The first laminated sheet 20 has a through hole 20a at a position facing the projection portion 11 of the cover member 10. With the through hole 20a, the sensor body 52 of the sensor unit 50 can be stored in the storage space S formed by the concave portion 11a on the inner surface of the cover member 10 and the through hole 20a without being obstructed by the first laminated sheet 20.

The first laminated sheet 20 includes a sticking layer 21 and a second adhesive layer 22 disposed on a surface on the cover member 10 side (in the +Z-axis direction) of the first laminated sheet 20.

### (Sticking layer)

As shown in FIG. 3, the sticking layer 21 includes a porous substrate 211 and a first adhesive layer 212 disposed on the living body side (-Z-axis direction) of the porous substrate 211.

### ((Porous substrate))

The porous substrate 211 has a porous structure and can be formed of a porous body having flexibility, waterproof property, and moisture permeability. For example, a foamed material having an open-cell structure, a closed-cell structure, a semi-closed-cell structure, or the like can be used for the porous body. Therefore, water vapor emitted/generated by perspiration or the like from the skin 2, to which the biological sensor 1 is affixed, can be discharged to the outside of the biological sensor 1 through the porous substrate 211.

The moisture permeability of the porous substrate 211 is preferably within a range from 100 g/m²·day to 5000 g/m²·day, more preferably within a range from 1000 g/m²·day to 4500 g/m²·day, and even more preferably within a range from 2000 g/m²·day to 4100 g/m²·day. When the moisture permeability of the sticking layer 21 is set to be within a range from 100 g/m²·day to 5000 g/m²·day, water vapor entering from one side of the porous substrate 211 can be caused to pass through the porous substrate 211 and can be stably discharged from the other side of the porous substrate 211.

For the material forming the porous substrate 211, a thermoplastic resin, such as a polyurethane resin, a polystyrene resin, a polyolefin resin, a silicone resin, an acrylic resin, a vinyl chloride resin, or a polyester resin, may be used.

The thickness of the porous substrate 211 is within a range from 0.5 mm to 1.5 mm.

The porous substrate 211 has a through hole 211a at a position facing the projection portion 11 of the cover member 10. Because the first adhesive layer 212 and the second adhesive layer 22 are formed on the surface of the porous substrate 211 other than the through hole 211a, the through hole 20a can be formed.

### ((First adhesive layer))

As shown in FIG. 3, the first adhesive layer 212 is affixed to the lower surface of the porous substrate 211, and has a function of sticking the second substrate 41 onto the porous substrate 211 and sticking the electrodes 30 onto the porous substrate 211.

The first adhesive layer 212 preferably has a moisture permeability. The water vapor or the like generated from the skin 2, to which the biological sensor 1 is affixed, can be discharged to the porous substrate 211 through the first adhesive layer 212. Furthermore, since the porous substrate 211 has a cell structure as described above, water vapor can be discharged to the outside of the biological sensor 1 via the second adhesive layer 22. Thus, it is possible to prevent perspiration or water vapor from accumulating at the interface between the skin 2, to which the biological sensor 1 is affixed, and the third adhesive layer 42. As a result, it is possible to prevent the biological sensor 1 from peeling off from the skin 2 due to the moisture accumulated at the interface between the skin 2 and the first adhesive layer 212 that reduces the adhesion force of the first adhesive layer 212.

The moisture permeability of the first adhesive layer 212 is preferably 1 g/m²·day or more, and more preferably 10 g/m²·day or more. Moreover, the moisture permeability of the first adhesive layer 212 is 10000 g/m²·day or less. If the moisture permeability of the first adhesive layer 212 is 10 g/m²·day or more, when the third adhesive layer 42 is affixed to the skin 2, perspiration or the like transmitted from the second laminated sheet 40 can be discharged to the outside, so that a load of the skin 2 can be reduced.

A material forming the first adhesive layer 212 preferably has a pressure-sensitive adhesiveness. The same material for the third adhesive layer 42 can be used. Specifically, an acrylic-based pressure-sensitive adhesive is preferably used.

The first adhesive layer 212 may be a double-sided adhesive tape formed of the above-described material. When the cover member 10 is laminated on the first adhesive layer 212 to form the biological sensor 1, the waterproof property of the biological sensor 1 can be enhanced and a bonding strength with the cover member 10 can be increased.

The first adhesive layer 212 may have a corrugated pattern (web pattern) formed on the surface in which an adhesive forming portion with the adhesive and an adherend portion without the adhesive are alternately formed. For the first adhesive layer 212, for example, a double-sided adhesive tape having a web pattern formed on the surface may be used. Since the first adhesive layer 212 has a web pattern on the surface, the adhesive can be attached to a convex portion of the surface and its periphery without the adhesive attaching to a concave portion of the surface and its periphery. Thus, since there are both a portion in which the adhesive is present on the surface of the first adhesive layer 212 and a portion in which the adhesive is not present, the adhesive can be dispersed on the surface of the first adhesive layer 212. The moisture permeability of the first adhesive layer 212 is likely to be higher, as the adhesive becomes thinner. Therefore, since the first adhesive layer 212 has a web pattern formed on the surface and a portion in which the adhesive is a partially thin, the moisture permeability can be enhanced while maintaining the adhesive strength, compared to the case where the web pattern is not formed.

Widths of the adhesive forming portion and the adherend portion can be suitably designed. The width of the adhesive forming portion is preferably, for example, within a range from 500 um to 1000 um, and the width of the adherend portion is preferably within a range from 1500 um to 5000 um. If the widths of the adhesive forming portion and the adherend portion are within the above-described corresponding preferred ranges, the first adhesive layer 212 exhibits an excellent moisture permeability while maintaining the adhesive strength.

The thickness of the first adhesive layer 212 is appropriately set. The thickness is within a range from 10 um to 300 um, more preferably within a range from 50 um to 200 um, and even more preferably within a range from 70 um to 110 um. If the thickness of the first adhesive layer 212 is within a range from 10 um to 300 um, the biological sensor 1 can be made thinner.

### (Second adhesive layer)

As shown in FIG. 3, the second adhesive layer 22 is disposed in a state of being affixed to the upper surface of the porous substrate 211. The second adhesive layer 22 is affixed to the upper surface of the porous substrate 211 at a position corresponding to the flat surface on the sticking side (-Y-axial direction) of the cover member 10, and has a function of sticking the cover member 10 onto the porous substrate 211.

For the material forming the second adhesive layer 22, a silicon-based adhesive, silicone-tape, or the like may be used.

The thickness of the second adhesive layer 22 may be appropriately set. The thickness is, for example, within a range from 10 um to 300 um.

### (Electrode)

As shown in FIG. 3, the electrode 30 is affixed to the lower surface that is the sticking side of the first adhesive layer 212 (in the -Z-axis direction) with a portion on the sensor body 52 side of the electrode 30 being connected to wirings 53a and 53b and the portion being held between the first adhesive layer 212 and the fourth adhesive layer 43. A portion of the electrode 30 that is not held between the first adhesive layer 212 and the fourth adhesive layer 43 is brought into contact with the living body. When the biological sensor 1 is affixed to the skin 2, the electrode 30 is brought into contact with the skin 2, so that the biological signal is detected. A biological signal is, for example, an electrical signal representing an electrocardiogram, an electroencephalogram, a pulse, or the like. The electrode 30 may be embedded in the second substrate 41 in a state of being exposed contactably with the skin 2.

The electrode 30 can be formed using an electrode sheet which is obtained by forming a cured product of a conductive composition including a conductive polymer and a binder resin, metals, alloys, or the like into a shape of sheet.

For the conductive polymer, for example, a polythiophene-based conductive polymer, a polyaniline-based conductive polymer, a polypyrrolebased conductive polymer, a polyacetylene-based conductive polymer, a polyphenylene-based conductive polymer and derivatives thereof, and a complex thereof may be used. The above-described conductive polymers may be used singly, or a combination of two or more conductive polymers may be used. Among them, a complex obtained by doping polyaniline as a dopant to polythiophene is preferably used. Among the complexes of polythiophene and polyaniline, PEDOT/PSS obtained by doping polystyrene sulfonic acid (poly4-styrene sulfonate; PSS) to poly3,4-ethylene dioxythiophene (PEDOT), is more preferably used because of a lower contact impedance with the living body and the high electrical conductivity.

The electrode 30 has a plurality of through holes 31 on the contact surface with the skin 2. Because the first adhesive layer 212 can be exposed to the sticking side through the through holes 31 in the state where the electrode 30 is affixed to the first adhesive layer 212, adhesiveness of the electrode 30 with the skin 2 can be enhanced.

### [Second laminated sheet]

As shown in FIG. 3, the second laminated sheet 40 includes a second substrate 41, a third adhesive layer 42, and a fourth adhesive layer 43.

### (Second substrate)

As shown in FIG. 3, an outer shape of the second substrate 41 on both sides, in the width direction (the X-axis direction) of the third adhesive layer 42, is substantially the same as an outer shape of the first laminated sheet 20 and the cover member 10 on both sides in the width direction (the X-axis direction). The length (Y-axis direction) of the second substrate 41 is shorter than the length (Y-axis direction) of the cover member 10 and the first laminated sheet 20. Both ends in the longitudinal direction of the second laminated sheet 40 are at positions where the wirings 53a and 53b of the sensor unit 50 are held between the second laminated sheet 40 and the first laminated sheet 20 and where the second laminated sheet 40 overlaps with the portion of the electrode 30. The fourth adhesive layer 43 is disposed on an upper surface of the second substrate 41, and the first adhesive layer 212 is disposed on the sticking surface of the first laminated sheet 20. The fourth adhesive layer 43 of the second laminated sheet 40 and the first adhesive layer 212 of the first laminated sheet 20 extending from both ends in the longitudinal direction of the second laminated sheet 40 form a sticking surface to the skin 2. Thus, water resistance/moisture permeability differs depending on the position on the sticking surface, and likewise adhesiveness differs. However, as a whole of the biological sensor 1, the adhesiveness on the sticking surface corresponding to the first laminated sheet 20 significantly affects a sticking performance to the skin 2.

The second substrate 41 can be formed of a flexible resin with appropriate elasticity, flexibility, and toughness. For materials forming the second substrate 41, for example, thermoplastic resins including a polyester-based resin, such as polyethylene terephthalate (PET), polybutylene terephthalate (PBT), polytrimethylene terephthalate, polyethylene naphthalate, and polybutylene naphthalate; an acrylic-based resin, such as polyacrylic acid, polymethacrylic acid, polymethyl acrylate, polymethyl methacrylate (PMMA), polyethyl methacrylate, and polybutyl acrylate; a polyolefinbased resin, such as polyethylene and polypropylene; a polystyrene-based resin, such as polystyrene, imide-modified polystyrene, acrylonitrile-butadiene styrene (ABS) resin, imide-modified ABS resin, styrene-acrylonitrile copolymerization (SAN) resin, and acrylonitrile-ethylene-propylene-diene styrene (AES) resin; a polyimide-based resin; a polyurethane-based resin; a silicone-based resin; and a polyvinyl chloride-based resin, such as polyvinyl chloride resin, and vinyl chloride-vinyl acetate copolymer resin, may be used. Among them, a polyolefin resin and PET are preferably used. The above-described thermoplastic resins are waterproof (with low moisture permeability). Thus, when the second substrate 41 is formed of the above-described thermoplastic resins, it is possible to prevent water vapor emitted/generated by perspiration from the skin 2 from entering the flexible substrate 51 side of the sensor unit 50 through the second substrate 41 in the state where the biological sensor 1 is affixed to the skin 2 of the living body.

Preferably, the second substrate 41 is formed in a flat plate shape, since the sensor unit 50 is disposed on the upper surface of the second substrate 41.

The thickness of the second substrate 41 may be appropriately selected. For example, the thickness is preferably within a range from 1 um to 300 um, more preferably within a range from 5 um to 100 um, and even more preferably within a range from 10 um to 50 um.

### (Third adhesive layer)

As shown in FIG. 3, the third adhesive layer 42 is disposed on the surface of the sticking side (in the -Z-axis direction) of the second substrate 41. The third adhesive layer 42 is brought into contact with the living body.

The third adhesive layer 42 preferably has pressure-sensitive adhesiveness. Since the third adhesive layer 42 has the pressure-sensitive adhesiveness, the biological sensor 1 can be readily affixed to the skin 2 by pressing the biological sensor 1 against the skin 2 of the living body.

The material of the third adhesive layer 42 is not particularly limited, as long as the material has a pressure-sensitive adhesiveness. The material includes a biocompatible material, and the like. Suitable materials forming the third adhesive layer 42 include, for example, an acrylic pressure-sensitive adhesive, and a silicone pressure-sensitive adhesive. The material preferably includes an acrylic pressure-sensitive adhesive.

The acrylic pressure-sensitive adhesive preferably includes an acrylic polymer as a main ingredient. The acrylic polymer can function as a pressure sensitive adhesive component. For the acrylic polymer, a polymer containing (meth)acrylic ester, such as isononyl acrylate or methoxyethyl acrylate, as a main ingredient, and obtained by being polymerized with a monomer component containing, as an optional component, a monomer that can be copolymerized with (meth)acrylic ester, such as acrylic acid, may be used

Preferably, the acrylic pressure-sensitive adhesive further includes a carboxylic acid ester. The carboxylic acid ester functions as a pressure-sensitive adhesive force regulator to reduce a pressure-sensitive adhesive force of the acrylic polymer to adjust the pressure-sensitive adhesive force of the third adhesive layer 42. For the carboxylic ester, carboxylic acid ester compatible with acrylic polymers may be used. For the carboxylic acid ester, trifatty acid glyceryl or the like may be used.

The acrylic pressure-sensitive adhesive may contain a crosslinking agent, as necessary. The crosslinking agents are cross-linking components that cross-link acrylic polymers. Suitable crosslinking agents include, for example, a polyisocyanate compound (a polyfunctional isocyanate compound), an epoxy compound, a melamine compound, a peroxide compound, a urea compound, a metal alkoxide compound, a metal chelate compound, a metal salt compound, a carbodiimide compound, an oxazoline compound, an aziridine compound, and an amine compound. Among the above-described compounds, the polyisocyanate compound is preferable. The above-described crosslinking agents may be used singly, or a combination of two or more crosslinking agents may be used.

The third adhesive layer 42 preferably has excellent biocompatibility. For example, when the third adhesive layer 42 is subjected to a keratin peeling test, a keratin peeling area ratio is preferably within a range from 0% to 50%, and more preferably within a range from 1% to 15%. When the keratin peeling area ratio is within the range of 0% to 50%, the load on the skin 2 can be suppressed even when the third adhesive layer 42 is affixed to the skin 2.

The third adhesive layer 42 is preferably moisture permeable. With the moisture permeability, it is possible to discharge water vapor or the like generated from the skin 2, to which the biological sensor 1 is affixed, to the first laminated sheet 20 side, through the third adhesive layer 42. Furthermore, since the first laminated sheet 20 has a cell structure which will be described later, water vapor can be discharged to the outside of the biological sensor 1 through the third adhesive layer 42. Therefore, it is possible to prevent perspiration or water vapor from accumulating at the interface between the skin 2, to which the biological sensor 1 is affixed, and the third adhesive layer 42. As a result, it is possible to prevent the biological sensor 1 from peeling off from the skin due to a decrease in the adhesion force of the third adhesive layer 42 by moisture accumulated at the interface between the skin 2 and the third adhesive layer 42.

The moisture permeability of the third adhesive layer 42 is preferably 300 g/m²·day or more, more preferably 600 g/m²·day or more, and even more preferably 1000 g/m²·day or more. Moreover, the moisture permeability of the third adhesive layer 42 is 10000 g/m²·day or less. If the moisture permeability of the third adhesive layer 42 is 300 g/m²·day or more, perspiration or the like generated from the skin 2 can be transmitted appropriately from the second substrate 41 to the outside even when the third adhesive layer 42 is affixed to the skin 2, thereby the load to the skin 2 can be reduced.

The thickness of the third adhesive layer 42 can be appropriately selected. The thickness is preferably within a range from 10 um to 300 um. When the thickness of the third adhesive layer 42 is within a range from 10 µm to 300 µm, the biological sensor 1 can be made thinner.

### (Fourth adhesive layer)

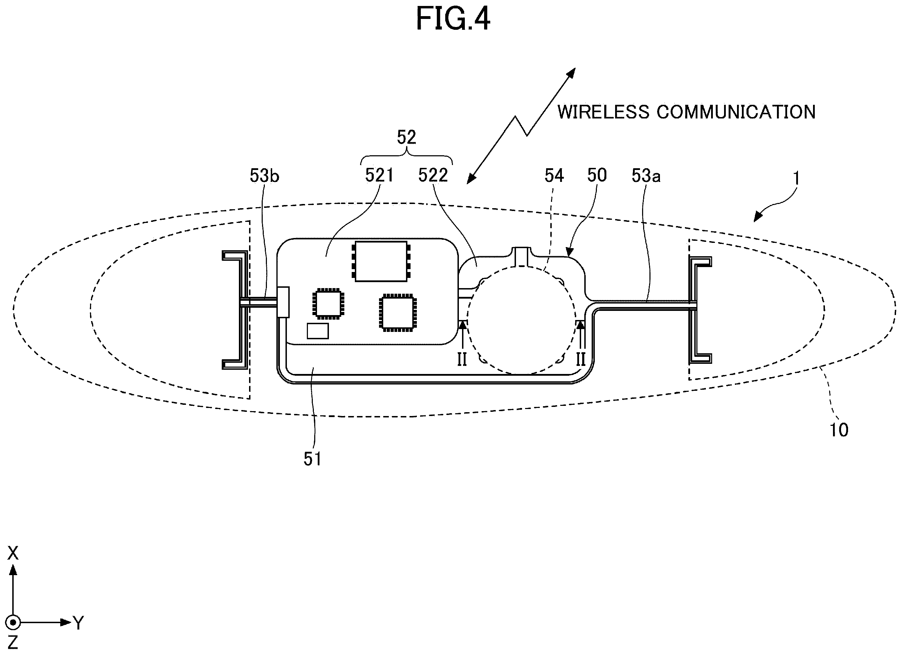

As shown in FIG. 4, the fourth adhesive layer 43 is disposed on the upper surface of the second substrate 41 on the cover member 10 side (in the +Z-axis direction), and is a layer to which the sensor unit 50 is affixed. Since for the fourth adhesive layer 43, a material the same as or similar to the third adhesive layer 42 can be used, details thereof will be omitted. The fourth adhesive layer 43 need not necessarily be provided, but may not be provided.

### (Sensor unit)

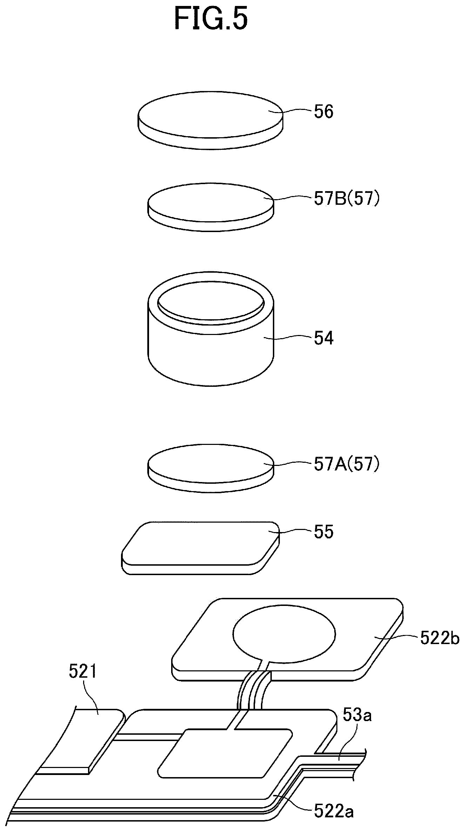

FIG. 4 is a plan view illustrating a configuration of the sensor unit 50, and FIG. 5 is an exploded perspective view of a part of the sensor unit 50. The dashed line in FIG. 4 represents the outer shape of the cover member 10. As shown in FIGS. 4 and 5, the sensor unit 50 includes a flexible substrate 51 on which various components for acquiring biological information are mounted, a sensor body 52, wirings 53a and 53b connected to the sensor body 52 in the longitudinal direction, a battery 54, a positive electrode pattern 55, a negative electrode pattern 56, and a conductive adhesive tape 57. Between a pad portion 522a and a pad portion 522b of the sensor unit 50, the positive electrode pattern 55, the conductive adhesive tape 57, the battery 54, the conductive adhesive tape 57, and the negative electrode pattern 56 are laminated in this order from the pad portion 522a side to the pad portion 522b side. In the present embodiment, the positive terminal of the battery 54 is set to be in the -Z-axis direction and the negative terminal is set to be in the +Z-axis direction. However, the positive terminal and the negative terminal may be reversed, i.e. the positive terminal may be in the +Z-axis direction and the negative terminal may be in the -Z-axis direction.

The flexible substrate 51 is made of a resin, and the flexible substrate 51 is integrally formed with the sensor body 52 and the wirings 53a and 53b.

An end of each of the wirings 53a and 53b is connected to electrode 30, as shown in FIG. 3. As shown in FIG. 4, the other end of the wiring 53a is connected to a switch or the like mounted to the component mounting unit 521 along the outer periphery of the sensor body 52. The other end of the wiring 53b is connected to a switch or the like mounted on the component mounting unit 521 in the same manner as the wiring 53a. The wirings 53a and 53b may be formed on any of wiring layers on the front surface side and the rear surface side of the flexible substrate 51.

As shown in FIG. 4, the sensor body 52 includes a component mounting unit 521 that is a controller and includes a battery mounting unit 522.

The component mounting unit 521 includes various components mounted on the flexible substrate 51, such as a CPU and an integrated circuit for processing biological signals acquired from a living body to generate biological signal data; a switch for activating the biological sensor 1; a flash memory for storing the biological signals; or a light emitting element. Examples of circuits using various components will be omitted. The component mounting unit 521 is operated by power supplied from the battery 54 mounted on the battery mounting unit 522.

The component mounting unit 521 wiredly or wirelessly communicates with an external device such as an operation checking device for checking an initial operation, or a readout device for reading biological information from the biological sensor 1.

The battery mounting unit 522 supplies power to the integrated circuit mounted on the component mounting unit 521. The battery 54 is mounted on the battery mounting unit 522, as shown in FIG. 2.

As shown in FIG. 5, the battery mounting unit 522 is disposed between the wiring 53a and the component mounting unit 521, and includes the pad portions 522a and 522b and a constriction portion 522c.

As shown in FIG. 5, the pad portion 522a is provided between the wiring 53a and the component mounting unit 521, located on the positive terminal side of the battery 54, and has the positive electrode pattern 55 to which the positive terminal is connected.

As shown in FIG. 5, the pad portion 522b is provided separated from the pad portion 522a by a predetermined distance along a direction orthogonal to the longitudinal direction (in the vertical direction in FIG. 3) with respect to the pad portion 522a. The pad 522b is located on the negative terminal (second terminal) side of the battery 54 and has the negative electrode pattern 56 to which the negative terminal is connected.

As shown in FIG. 5, the constriction portion 522c is disposed between the pad portions 522a and 522b to connect the pad portions 522a and 522b to each other.

As shown in FIG. 5, the battery 54 is arranged between the positive and negative electrode patterns 55 and 56. The battery 54 has positive and negative terminals. For the battery 54, a publicly-known battery may be used. The battery 54 may be a coin-type battery, such as CR2025.

As shown in FIG. 5, the positive electrode pattern 55 is located on the positive terminal side of the battery 54 and is connected to the positive terminal. The positive electrode pattern 55 has a rectangular shape with chamfered corners.

As shown in FIG. 5, the negative electrode pattern 56 is located on the negative terminal side of the battery 54 and is connected to the negative terminal. The negative electrode pattern 56 has a shape substantially corresponding to the size of the circular shape of the negative terminal of the battery 54. The diameter of the negative electrode pattern 56 is, for example, equal to the diameter of the battery 54 and approximately equal to the diagonal length of the positive electrode pattern 55.

The conductive adhesive tape 57 is a conductive adhesive that is disposed between the battery 54 and the positive electrode pattern 55 and also is disposed between the battery 54 and the negative electrode pattern 56. The conductive adhesive tape may also generally be referred to as a conductive adhesive sheet, a conductive adhesive film, or the like.

A conductive adhesive tape 57A and a conductive adhesive tape 57B are affixed to the entire positive electrode pattern 55 and the negative electrode pattern 56, respectively, when a battery 54 is mounted to the biological sensor 1. Then, the positive terminal and the negative terminal of the battery 54 are affixed to the positive electrode pattern 55 and the negative electrode pattern 56 via the conductive adhesive tape 57A and the conductive adhesive tape 57B, respectively, so that the battery 54 is mounted to the battery mounting unit 522. FIG. 4 shows the sensor body 52 in which the battery 54 is mounted to the battery mounting unit 522 in the state where the battery 54 is held between the positive electrode pattern 55 and the negative electrode pattern 56 by deflecting the constriction portion 522c.

As shown in FIG. 3, a peelable sheet 60 is preferably affixed to the surface of the biological sensor 1 on the sticking side (-Z-axis direction) until the biological sensor 1 is affixed to the skin 2, in order to protect the second substrate 41 and the electrode 30. By peeling the peelable sheet 60 off the second substrate 41 and the electrodes 30 when the biological sensor 1 is used, the adhesion force of the second substrate 41 can be maintained.

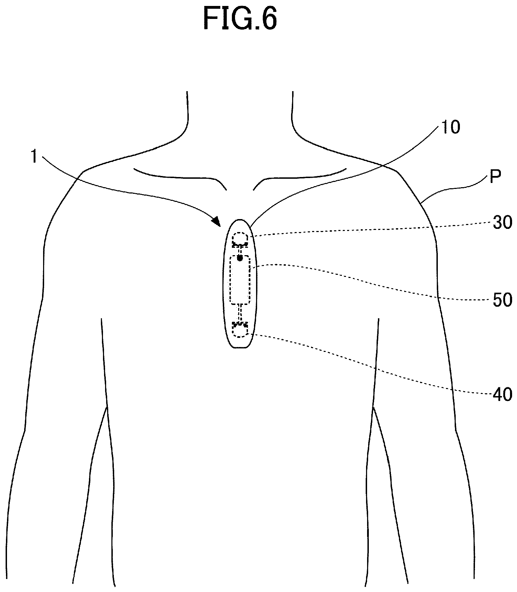

FIG. 6 is an explanatory diagram illustrating a state where the biological sensor 1 shown in FIG. 1 is affixed to a chest of the living body P. For example, the longitudinal direction (Y-axis direction) of the biological sensor 1 is aligned with the sternum of the living body P, and the biological sensor 1 is affixed to the skin of the living body P with one electrode 30 being on the upper side and another electrode 30 being on the lower side of the living body P. The biological sensor 1 acquires a biological signal, such as an electrocardiogram signal, through the electrodes 30 from the living body P in the state where the electrodes 30 are pressed into contact with the skin of the living body P by sticking the third adhesive layer 42 shown in FIG. 2 onto the skin of the living body P. The biological sensor 1 stores the acquired biological signal data in a non-volatile memory such as a flash memory mounted in the component mounting unit 521.

As described above, the biological sensor 1 includes the cover member 10, the porous substrate 211, and exhibits a shear stress of from 5×10⁴ N/m² to 65×10⁴ N/m² when the sticking layer 21, having the porous substrate 211 and the first adhesive layer 212, is deformed in a direction perpendicular to the thickness direction of the sticking layer 21 (X-axis direction or Y-axis direction) by 5% to 15% of a length of the sticking layer 21, and a moisture permeability of the sticking layer 21 is within a range from 65 g/m²·day to 4000 g/m²·day. With the above-described properties, it is possible to soften the sticking layer 21 by increasing the shear stress when the sticking layer 21 is deformed, while increasing an air-permeability by controlling the moisture permeability to be within a predetermined range, and thereby the entire sticking layer 21 has adequate flexibility. As a result, upon attaching the biological sensor 1 to the skin 2, even when the skin 2 is stretched due to attaching the biological sensor 1 on the skin 2 with pressure, a body movement, or the like, it is possible to reduce the stress generated at the interface between the third adhesive layer 42, which is provided on the surface of the second substrate 41 on the sticking side (-Z-axis direction), and the skin 2. Thus, it is possible to prevent the biological sensor 1 from peeling off the skin 2. Therefore, the biological sensor 1 can be stably affixed to the skin 2.

In particular, in the biological sensor 1 having the above-described configuration, since the electrode 30 is disposed on a part of the sticking surface of the first adhesive layer 212, and the porous substrate 211 has a through hole 211a at a substantially central portion thereof, it is important that the first adhesive layer 212 readily follows the movement of the skin 2, and that the biological sensor 1 is flexible. In the biological sensor 1, when a shear force applied to the sticking layer 21 is within a predetermined range, the sticking layer 21 is softened by increasing a shear stress when the sticking layer 21 is deformed, while increasing an air-permeability of the sticking layer 21 by controlling the moisture permeability to be within a predetermined range, and thereby the entire sticking layer 21 has adequate flexibility. Thus, it is possible to prevent the sticking surface of the first adhesive layer 212, onto which the electrodes 30 are affixed, from peeling off from the skin 2. Furthermore, in the planar view of the biological sensor 1, it is possible to prevent the sticking surface located in the region of the porous substrate 211, including the through hole 211a and the connecting portions of the wirings 53a and 53b to the electrode 30, from peeling off from the skin 2.

Accordingly, the biological sensor 1 can stably measure biological information from the skin 2, since at least a part of the biological sensor 1 can be prevented from peeling off the subject's skin 2 even if the subject moves during using the biological sensor 1.

The biological sensor 1 may include a second adhesive layer 22 on a surface of the sticking layer 21 on the cover member 10 side that is an upper surface of the sticking layer 21. According to the above-described configuration, the first laminated sheet 20 can be made softer. Thus, when the skin 2 is stretched due to the body movement, the first adhesive layer 212 and the third adhesive layer 42 are more readily deformed along the interface with the skin 2, and the stress generated at the interface between the first adhesive layer 212 and the third adhesive layer 42 and the skin 2 can be reduced more. Accordingly, since the biological sensor 1 is further prevented from peeling off from the skin 2, it is possible to maintain the stable state of sticking to the skin 2.

Additionally, a hardness of the cover member 10 of the biological sensor 1 can be within a range from 40 to 70. When the hardness of the cover member 10 is within a range from 40 to 70, the cover member 10 can have an appropriate softness, and it is possible to reduce the obstructing the deformation of the second laminated sheet 40 by the cover member 10. Thus, because when the skin 2 is stretched by the body movement the third adhesive layer 42 can be more readily deformed along the interface with the skin 2, the stress at the interface between the third adhesive layer 42 and the skin 2 can be further reduced. Accordingly, since the biological sensor 1 can be more stably prevented from being peeled from the skin 2, it is possible to more stably maintain the state of sticking to the skin 2.

The moisture permeability of the porous substrate 211 of the biological sensor 1 can be within a range from 100 g/m²·day to 5000 g/m²·day. Accordingly, the porous substrate 211 can stably discharge water vapor generated from the skin 2 to the outside of the biological sensor 1 via the first adhesive layer 212 and the second adhesive layer 22, and thus it is possible to further suppress the peeling from the skin 2.

Moreover, the biological sensor 1 can exhibit the shear stress of from 5×10⁴ N/m² to 25×10⁴ N/m² when 25% to 35% of the entire length of the biological sensor 1 (in the Y-axis direction) with respect to the contact surface with the skin 2 is deformed. Typically, when a biological sensor is affixed to skin, an amount of deformation of the biological sensor with respect to a contact surface with the skin 2 is 20% or less of the entire length of the biological sensor. Even when the biological sensor 1 is deformed by 25% to 35% of the entire length of the biological sensor 1, the shear stress of the biological sensor 1 can be made within a range from 5×10⁴ N/m² to 25×10⁴ N/m². Thus, in the state where the biological sensor 1 is affixed to the skin 2, even when the skin 2 is stretched by the body movement, it is possible to more stably prevent the biological sensor 1 from peeling off from the skin 2. It is possible to maintain more stably the state of being affixed to the skin 2.

The biological sensor 1 includes the electrode 30, the second substrate 41, and a sensor body 52. The cover member 10 has the concave portion 11a on the skin 2 side. The porous substrate 211 has the through hole 211a in a position corresponding to the concave portion 11a, and the storage space S can be formed by the concave portion 11a and the through hole 211a. Even if the biological sensor 1 is provided with the sensor body 52 inside the biological sensor 1, the first adhesive layer 212 can further suppress the peeling from the skin 2, and the biological sensor 1 can maintain the state of stably sticking to the skin 2.

The biological sensor 1 includes the third adhesive layer 42, and can form a sticking surface to the living body by the first adhesive layer 212 and the third adhesive layer 42. In the biological sensor 1, Even when the third adhesive layer 42 is in contact with the skin 2, the third adhesive layer 42 can further suppress the peeling from the skin 2, and the biological sensor 1 can maintain the state where the third adhesive layer 42 is stably affixed to the skin 2.

In addition, the biological sensor 1 may provide a through hole 31 in the electrode 30. By exposing the first adhesive layer 212 through the through hole 31 to the sticking side, the adhesion between the electrode 30 and the skin 2 can be enhanced. Therefore, even when the electrode 30 is affixed to the first adhesive layer 212, the biological sensor 1 can prevent the first adhesive layer 212 from peeling off from the skin 2, and can maintain the state of stably sticking to the skin 2.

As described above, because the biological sensor 1 can make it unlikely to peel off from the skin 2, the biological sensor 1 may be suitably used for a wearable device for healthcare, such as a biological sensor.

### [Example]

In the following, the embodiments will be more specifically described presenting practical examples and comparative examples. However, the embodiments are not limited by the practical examples and comparative examples.

### <Example 1>

### [Preparation of biological sensor]

### (Preparation of first laminated sheet)

A first adhesive layer (long-term adhesive tape 1 (by Nitto Denko Corporation, thickness: 70 um)) was formed on a lower surface of a porous substrate 1 (polyolefin foam sheet (Folec(TM)), by INOAC Corporation, thickness: 0.5 mm), formed in a rectangular shape. The long-term adhesive tape 1 was a double-sided adhesive tape having a corrugated pattern (web pattern) formed on the surface thereof such that a width of an adhesive agent forming portion without an adhesive agent was about 500 um and a width of an adherend portion without an adhesive agent was about 1500 um. Thereafter, a second adhesive layer (a silicone tape 1 (ST503(HC)60, by Nitto Denko Corporation, thickness: 60 um) was formed on an upper surface of a sticking layer. Thus, the first laminated sheet was prepared.

### (Preparation of second laminated sheet)

A second laminated sheet, which was a skin tape obtained by sticking adhesive films 1 (Permerol, by Nitto Denko Corporation, moisture permeability: 21 g/m²·day), as a third adhesive layer, onto both surfaces of a substrate 1 (PET (PET-50-SCA1 (white), by Mitsui & Co. Plastics, Ltd.), thickness: 38 um), formed in a rectangular shape, was prepared.

### (Preparation of cover member)

A cover member was prepared by forming a coating layer with a Shore hardness A40 formed of a silicone rubber on a support formed using PET as a base resin, and forming the product in a predetermined shape.

### (Preparation of biological sensor)

A sensor unit provided with a battery and a controller was deposited in the center of an upper surface of the second laminated sheet. Then, a pair of electrodes were affixed to a sticking surface side of the first adhesive layer in the state of being held by the first adhesive layer of the first laminated sheet and the second laminated sheet, thereby the electrodes were connected to a wiring of the sensor unit. Thereafter, a cover member was laminated on the first laminated sheet so that the sensor unit was arranged within a storage space formed by the first laminated sheet and the cover member. Thus, the biological sensor was prepared.

### [Evaluation of moisture permeability of porous substrate]

The moisture permeability of the porous substrate 1 was measured according to the conditions of JIS Z 0208 (Moisture permeability test method of moisture-proof packaging material (cup method)). A test article was prepared from the porous substrate having a width of 5 cm × a length of 5 cm × a thickness of 0.5 mm, and a mass of the test article was measured. Then, the test article was left under a constant temperature and humidity environment with a temperature of 40°C and a relative humidity of 30% for 24 hours, and the mass of the test article was measured. The moisture permeability of the porous substrate 1 at a thickness of 500 um was calculated by using the following equation (1). Moistutre permeability of the porous substrate (g/m2·day) = ((mass before leaving) - (mass after leaving)) × 882.192

### [Evaluation of characteristics of sticking layer]

A shear stress when the sticking layer was deformed by 10%, moisture permeability, and water retention rate were evaluated as characteristics of the sticking layer.

### (Shear stress at 10% deformation)

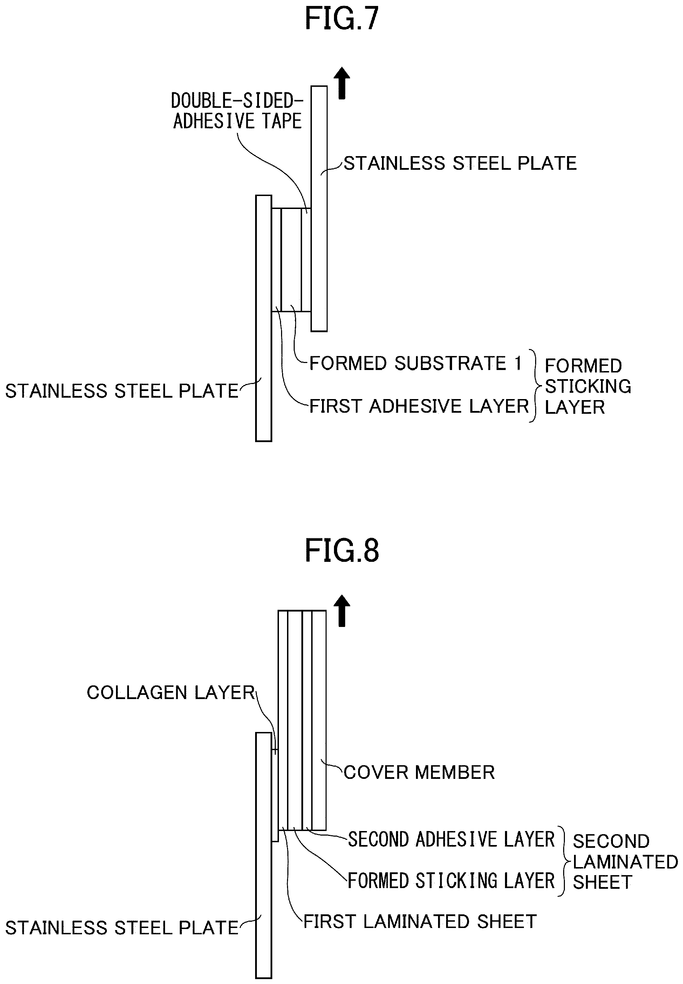

As shown in FIG. 7, a double-sided adhesive tape (No. 5000S, by Nitto Denko Corporation) was affixed to one surface of the sticking layer (1 cm × 1 cm) and then the sticking layer was held by a pair of stainless steel plates (SUS plates). Then, one stainless steel plate was pulled at a rate of 360 mm/min parallel to the other stainless steel plate, until the length of the sticking layer was deformed by 10% (i.e. 1.1 cm), and the shear stress when the adhesive layer was deformed by 10% in the length direction was measured.

### (Evaluation of moisture permeability and water retention rate)

The moisture permeability of the sticking layer was measured by the same method as the above-described method of the porous substrate 1. The water retention rate of the sticking layer was calculated by using the following equation (2). Water retention rate (%) of the sticking layer = ((mass before leaving) - (mass after leaving)) / ((mass before leaving) × 100)

### [Evaluation of moisture permeability of the second laminated sheet]

The moisture permeability of the second laminated sheet was measured by the same method as the above-described method for measuring moisture permeability of the porous substrate 1.

### [Evaluation of characteristics of biological sensor]

As characteristics of the biological sensor obtained as above, a shear stress when the biological sensor is deformed by 30% in the length direction (at 30% deformation of the biological sensor), stability of sticking, and a peel position were evaluated.

### (Evaluation of shear stress at 30% deformation)

As shown in FIG. 8, the biological sensor was regarded to be a laminated body including a cover member, a first laminated sheet, and a second laminated sheet, and a test piece was prepared from the laminated body having a width of 1 cm × a length of 4 cm. A test article was prepared by sticking an adhesion surface of the test piece onto a collagen membrane (Nippicasing #300, by Nippi Collagen Cosmetics, Ltd.) fixed to a stainless steel plate (SUS plate). Then, the test article was pulled at a range of 360 mm/min parallel to the stainless steel plate, until the length of the test article was deformed by 30%, and the shear stress when the test article was deformed by 30% in the length direction was measured.

### (Evaluation of stability of sticking and peeling position)

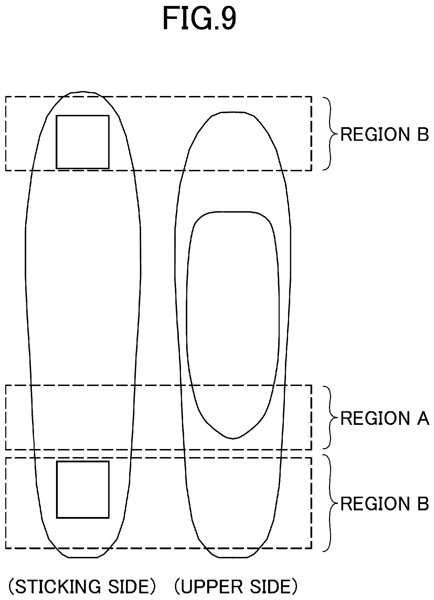

The stability of sticking of the biological sensor was evaluated by sticking the biological sensors onto skins of a plurality of men and women for 24 hours, respectively, and observing an occurrence of peeling and a position of the peeling. When the biological sensor was not peeled off from the skins of the plurality of men or women, the stability of sticking was evaluated to be excellent (symbol "A" in TABLE 1). When the biological sensor was peeled off from the skin of the plurality of men or women a few times, the stability was evaluated to be good (symbol "B" in TABLE 1). When the biological sensor was peeled off from the skins of all men or women, the stability of sticking of the biological sensor was evaluated to be poor (symbol "C" in TABLE 1). In addition, it was investigated whether a peeling position is within a region between the central portion of the adhesive layer and the electrode in a plan view of the biological sensor (region A in FIG. 9) or within a region in which the electrode is disposed in the plan view of the biological sensor (region B in FIG. 9) .

### <Example 2>

In Example 2, evaluation was performed in the same manner as Example 1, except that the thickness of the porous substrate 1 was changed, and a shear force at 10% deformation of the porous substrate 1 was changed in Example 1.

### <Examples 3 to 6>

In Examples 3 to 6, evaluation was performed in the same manner as Example 1, except that the thickness of the porous substrate 1 was changed, a shear force at 10% deformation of the porous substrate 1 was changed, and a type of the cover member was changed in Example 1.

### <Example 7>

In Example 7, evaluation was performed in the same manner as Example 1, except that the thickness of the porous substrate 1 was changed, a type of the second adhesive layer of the first laminated sheet was changed to a long-term adhesive tape 2, which will be described below, a shear force at deformation of the sticking layer was changed, and a type of the cover member was changed in Example 1. In addition, the long-term adhesive tape 2 was a double-sided adhesive tape, formed of the same adhesive agent as that of the long-term adhesive tape 1. However, a corrugated pattern was not formed on the surface of the long-term adhesive tape 2.

The second adhesive layer: a long-term adhesive tape 2 (by Nitto Denko Corporation) with thickness of 60 um.

### <Example 8>

In Example 8, evaluation was performed in the same manner as Example 1, except that the thickness of the porous substrate 1 was changed, and a type of a second adhesive agent of a sheet layer was changed to a long-term adhesive tape 3, which will be described below, a shear force at deformation of the sticking layer was changed in Example 1.

The second adhesive agent: a long-term adhesive tape 3 (SLY-25 by Nitto Denko Corporation) with thickness of 25 um.

### <Comparative example 1>

In Comparative example 1, evaluation was performed in the same manner as Example 1, except that the porous substrate 1 was not used.

### <Comparative example 2>

In Comparative example 2, evaluation was performed in the same manner as Example 1, except that the porous substrate 1 was changed to a porous substrate 2, and a type of the first adhesive layer on the lower surface of the second laminated sheet was changed to a long-term adhesive tape 2, which will be described below, and a shear force at deformation of the sticking layer was changed in Example 1.

### Porous substrate 2: Volara by Sekisui Chemical Co., Ltd. with thickness of 1 mm

Second adhesive agent: a long-term adhesive tape 2 (SLY-25 by Nitto Denko Corporation) with thickness of 35 um.

TABLE 1 shows types of cover members, configuration of the first laminated sheet, configuration of the second laminated sheet, and results of evaluation of the characteristics of the biological sensor in each of the Examples and Comparative examples.

**[TABLE 1]**

| | Cover member | First laminated sheet | | | | | | | Second laminated sheet | | | Biological sensor | | | |
|---|---|---|---|---|---|---|---|---|---|---|---|---|---|---|---|
| | Type | Sticking layer | | | | | | Second adhesive layer | Substrate (thickness ) | Third adhesive layer (thickness ) | Moisture permeability [g/m² ·day] | Shear force at 30% deformation [N/m²] | Stability of sticking | | Peeling position |
| | | Porous substrate | | First adhesive layer | Shear force at 10% deformation [N/m²] | Moisture permeability [g/m²·day] | Water retention rate [%] | | | | | | | | |
| | | Type (thickness ) | Moisture permeability [g/m²·day] | | | | | | | | | | women | men | |
| Ex.1 | Hardness 40/PET | Porous substrate 1 (0.5 mm) | 4067 | Long-term adhesive tape 1 | 14.6×10⁴ | 3891 | 17.0 | Silicone tape 1 | Substrate 1 (38µm) | Adhesive agent 1 (25 um) | 21 | 19.10×10⁴ | C | A | A |
| Ex.2 | Hardness 40/PET | Porous substrate 1 (1 mm) | 3659 | Long-term adhesive tape 1 | 11.1×10⁴ | 1660 | 7.6 | Silicone tape 1 | Substrate 1 (38 µm) | Adhesive agent 1 (25 um) | 21 | 13.02×10⁴ | B | A | A |
| Ex.3 | Hardness 40 | Porous substrate 1 (1 mm) | 3659 | Long-term adhesive tape 1 | 11.1×10⁴ | 1660 | 7.6 | Silicone tape 1 | Substrate 1 (38 µm) | Adhesive agent (25 um) | 21 | 5.73×10⁴ | B | A | A |
| Ex.4 | Hardness 50 | Porous substrate 1 (1 mm) | 3659 | Long-term adhesive tape 1 | 11.1×10⁴ | 1660 | 7.6 | Silicone tape 1 | Substrate 1 (38 µm) | Adhesive agent (25 um) | 21 | 6.97×10⁴ | C | A | A |
| Ex.5 | Hardness 60 | Porous substrate 1 (1 mm) | 3659 | Long-term adhesive tape 1 | 11.1×10⁴ | 1660 | 7.6 | Silicone tape 1 | Substrate 1 (38 µm) | Adhesive agent (25 um) | 21 | 7.13×10⁴ | C | A | B |
| Ex.6 | Hardness 70 | Porous substrate 1 (1 mm) | 3659 | Long-term adhesive tape 1 | 11.1×10⁴ | 1660 | 7.6 | Silicone tape 1 | Substrate 1 (38 µm) | Adhesive agent (25 um) | 21 | 8.18×10⁴ | C | A | B |
| Ex.7 | Hardness 40 | Porous substrate 1 (1 mm) | 3659 | Long-term adhesive tape 2 | 11.1×10⁴ | 863 | 9.5 | Silicone tape 1 | Substrate 1 (38 µm) | Adhesive agent (25 um) | 21 | 5.64×10⁴ | B | A | B |
| Ex.8 | Hardness 40/PET | Porous substrate 1 (1 mm) | 3659 | Long-term adhesive tape 3 | 11.1×10⁴ | 92.4 | 10.6 | Silicone tape 1 | Substrate 1 (38 µm) | Adhesive agent (25 µm) | 21 | 24.05×10⁴ | C | A | A |
| Comp. ex.1 | Hardness 40/PET | - | 55 | Long-term adhesive tape 3 | 64.0×10⁴ | 62 | 1.9 | Silicone tape 1 | Substrate 1 (38 µm) | Adhesive agent (25 µm) | 21 | 29.24×10⁴ | C | C | A |
| Comp. ex.2 | Hardness 40/PET | Porous substrate 2 (1 mm) | 76 | Long-term adhesive tape 2 | 14.2×10⁴ | 53 | 42 | Silicone tape 1 | Substrate 1 (38 µm) | Adhesive agent (25 um) | 21 | 15.87×10⁴ | C | C | A |

As shown in TABLE 1, in Examples 1 to 8, the shear stress was 15×10⁴ N/m² or less when the sticking layer was deformed by 10%, and the moisture permeability of the sticking layer was within a range from 92.4 g/m²·day to 3891 g/m²·day. On the other hand, in Comparative examples 1 and 2, the moisture permeability of the sticking layer was 76 g/m²·day or less.

Accordingly, different from the biological sensors in Comparative examples 1 and 2, the biological sensors in Examples 1 to 8 can flexibly respond to variations of the skin by setting the shear stress when the sticking layer is deformed by 10% to be 15×10⁴ N/m² or less, and setting the moisture permeability of the sticking layer to be within a range from 92.4 g/m²·day to 3891 g/m²·day, thereby enabling water vapor generated from the skin to be discharged to the outside. Thus, peeling from the skin can be suppressed. Accordingly, since the biological sensor according to the embodiment of the present application can be stably affixed to the skin, it is possible to stably detect electrical signals obtained from the living body with high sensitivity. Therefore, the biological sensor can be effectively used for stably measuring the electrocardiogram for a long period of time (e.g. 24 hours) in close contact with the subject's skin.

As described above, the embodiments of the present application have been described. However, the embodiments have been illustrated as examples, and the present invention is not limited to the embodiments.

The present international application claims the priority based on Japanese Patent Application No. 2020-059650, filed March 30, 2020.

### [Reference signs list]

- 1: Biological sensor
- 2: Skin
- 10: Cover member
- 20: First laminated sheet (first laminated body)
- 21: Sticking layer
- 211: Porous substrate
- 212: First adhesive layer
- 22: Second adhesive layer
- 30: Electrode
- 31: Through hole
- 40: Second laminated sheet (second laminated body)
- 41: Second substrate
- 42: Third adhesive layer
- 43: Fourth adhesive layer
- 50: Sensor unit
- 51: Flexible substrates (resin substrates)
- 52: Sensor body
- 54: Battery

## Claims

1. A biological sensor (1) that is to be affixed to a living body and is for acquiring a biological signal, the biological sensor comprising:
a cover member (10); and
a porous substrate (211) having a porous structure, the porous substrate being disposed on the cover member on a side of the living body,
wherein a sticking layer (21), including the porous substrate and a first adhesive layer (212) that is disposed on the porous substrate on a side of the living body, exhibits a shear stress of from 5×10⁴ N/m² to 65×10⁴ N/m² when the sticking layer is deformed in a direction perpendicular to a thickness direction of the sticking layer by 5% to 15% of a length of the sticking layer, wherein the thickness of the porous substrate is within a range from 0.5 mm to 1.5 mm and the thickness of the first adhesive layer is within a range from 10 um to 300 um,
wherein a moisture permeability of the sticking layer is within a range from 65 g/m²·day to 4000 g/m²·day, and wherein the biological sensor further comprises:
an electrode (30) affixed to the first adhesive layer;
a sensor body (52) that is connected to the electrode and acquires biological information; and
a second substrate (51) on which the sensor body is mounted,
wherein the cover member includes a concave portion (11a) formed in a recessed shape on a side of the living body,
wherein the porous substrate has a through hole (211a) at a position facing the concave portion, and
wherein the concave portion and the through hole form a storage space (S) for storing the sensor body.

2. The biological sensor according to claim 1,
wherein a second adhesive layer (22) is disposed on a surface on the sticking layer on a side of the cover member.

3. The biological sensor according to claim 1 or 2,
wherein a Shore A hardness of the cover member is within a range from 40 to 70.

4. The biological sensor according to any one of claims 1 to 3,
wherein a moisture permeability of the porous substrate is within a range from 100 g/m²·day to 5000 g/m² ·day.

5. The biological sensor according to any one of claims 1 to 4,
wherein the biological sensor exhibits a shear stress of from 5×10⁴ N/m² to 25×10⁴ N/m² when 25% to 35% of an entire length of the biological sensor is deformed with respect to a contact surface with the living body.

6. The biological sensor according to claim 1 to 5, further comprising:
a third adhesive layer (42) provided on the second substrate on a side of the living body,
wherein the third adhesive layer and the first adhesive layer form a sticking surface to be affixed to the living body.

7. The biological sensor according to claim 1 or 6,
wherein the electrode has a through hole (31) through which the first adhesive layer can be exposed in a state where the electrode is affixed to the first adhesive layer.

## Patentansprüche

1. Biologischer Sensor (1), der an einem lebenden Körper befestigt werden soll und zum Beziehen eines biologischen Signals vorgesehen ist, wobei der biologische Sensor umfasst:
ein Abdeckelement (10); und
ein poröses Substrat (211) mit einer porösen Struktur, wobei das poröse Substrat auf dem Abdeckelement auf einer Seite des lebenden Körpers angeordnet ist,
wobei eine Klebeschicht (21), einschließlich des porösen Substrats und einer ersten Klebstoffschicht (212), angeordnet auf dem porösen Substrat auf einer Seite des lebenden Körpers, eine Scherspannung von 5 × 10⁴ N/m² bis 65 × 10⁴ N/m² zeigt, wenn die Klebeschicht in einer Richtung rechtwinkelig zu einer Dickenrichtung der Klebeschicht um 5 % bis 15 % einer Länge der Klebeschicht verformt ist, wobei die Dicke des porösen Substrats in einem Bereich von 0,5 mm bis 1,5 mm liegt und die Dicke der ersten Klebstoffschicht in einem Bereich von 10 µm bis 300 µm liegt,
wobei eine Feuchtigkeitsdurchlässigkeit der Klebeschicht in einem Bereich von 65 g/m²·Tag bis 4000 g/m²·Tag liegt, und wobei der biologische Sensor weiter umfasst:
eine Elektrode (30), die an der ersten Klebstoffschicht befestigt ist;
einen Sensorkörper (52), der mit der Elektrode verbunden ist und biologische Informationen bezieht; und
ein zweites Substrat (51), auf dem der Sensorkörper angebracht ist,
wobei das Abdeckelement einen konkaven Abschnitt (11a) einschließt, der in einer vertieften Form auf einer Seite des lebenden Körpers ausgebildet ist,
wobei das poröse Substrat eine Durchgangsbohrung (211a) an einer dem konkaven Abschnitt zugewandten Position aufweist, und
wobei der konkave Abschnitt und die Durchgangsbohrung einen Lagerbereich (S) zum Lagern des Sensorkörpers bilden.

2. Biologischer Sensor nach Anspruch 1,
wobei eine zweite Klebstoffschicht (22) auf einer Fläche auf der Klebeschicht auf einer Seite des Abdeckelements angeordnet ist.

3. Biologischer Sensor nach Anspruch 1 oder 2,
wobei eine Shore A-Härte des Abdeckelements in einem Bereich von 40 bis 70 liegt.

4. Biologischer Sensor nach einem der Ansprüche 1 bis 3,
wobei eine Feuchtigkeitsdurchlässigkeit des porösen Substrats in einem Bereich von 100 g/m²·Tag bis 5000 g/m²·Tag liegt.

5. Biologischer Sensor nach einem der Ansprüche 1 bis 4,
wobei der biologische Sensor eine Scherspannung von 5 × 10⁴ N/m² bis 25 × 10⁴ N/m² zeigt, wenn 25 % bis 35 % einer Gesamtlänge des biologischen Sensors in Bezug auf eine Kontaktfläche mit dem lebenden Körper verformt sind.

6. Biologischer Sensor nach Anspruch 1 bis 5, weiter umfassend:
eine dritte Klebstoffschicht (42), die auf dem zweiten Substrat auf einer Seite des lebenden Körpers bereitgestellt ist,
wobei die dritte Klebstoffschicht und die erste Klebstoffschicht eine Klebefläche bilden, die an dem lebenden Körper befestigt werden soll.

7. Biologischer Sensor nach Anspruch 1 oder 6,
wobei die Elektrode eine Durchgangsbohrung (31) aufweist, durch welche die erste Klebstoffschicht in einem Zustand freigelegt werden kann, in dem die Elektrode an der ersten Klebstoffschicht befestigt ist.

## Revendications

1. Capteur biologique (1) qui doit être fixé sur un corps vivant et est destiné à acquérir un signal biologique, le capteur biologique comprenant :
un élément de couvercle (10) ; et
un substrat poreux (211) ayant une structure poreuse, le substrat poreux étant disposé sur l'élément de couvercle sur un côté du corps vivant,
dans lequel une couche de fixation (21), incluant le substrat poreux et une première couche adhésive (212) qui est disposée sur le substrat poreux sur un côté du corps vivant, présente une contrainte de cisaillement de 5×l0⁴ N/m² à 65×l0⁴ N/m² lorsque la couche de fixation est déformée dans une direction perpendiculaire à une direction de l'épaisseur de la couche de fixation de 5 % à 15 % d'une longueur de la couche de fixation, dans lequel l'épaisseur du substrat poreux est dans une plage allant de 0,5 mm à 1,5 mm et l'épaisseur de la première couche adhésive est dans une plage allant de 10 µm à 300 µm,
dans lequel une perméabilité à l'humidité de la couche de fixation est dans une plage allant de 65 g/m²·jour à 4000 g/m²·jour, et dans lequel le capteur biologique comprend en outre :
une électrode (30) fixée à la première couche adhésive ;
un corps de capteur (52) qui est connecté à l'électrode et acquiert des informations biologiques ; et
un second substrat (51) sur lequel le corps de capteur est monté,
dans lequel l'élément de couvercle inclut une partie concave (11a) formée en creux sur un côté du corps vivant,
dans lequel le substrat poreux a un trou traversant (211a) au niveau d'une position faisant face à la partie concave, et
dans lequel la partie concave et le trou traversant forment un espace de stockage (S) pour stocker le corps de capteur.

2. Capteur biologique selon la revendication 1,
dans lequel une deuxième couche adhésive (22) est disposée sur une surface de la couche de fixation sur un côté de l'élément de couvercle.

3. Capteur biologique selon la revendication 1 ou la revendication 2,
dans lequel une dureté Shore A de l'élément de couvercle est dans une plage allant de 40 à 70.

4. Capteur biologique selon l'une quelconque des revendications 1 à 3,
dans lequel une perméabilité à l'humidité du substrat poreux est dans une plage allant de 100 g/m²·jour à 5000 g/m²·jour.

5. Capteur biologique selon l'une quelconque des revendications 1 à 4,
dans lequel le capteur biologique présente une contrainte de cisaillement allant de 5×10⁴ N/m² à 25×10⁴ N/m² lorsque 25 % à 35 % d'une longueur totale du capteur biologique est déformée par rapport à une surface de contact avec le corps vivant.

6. Capteur biologique selon les revendications 1 à 5, comprenant en outre :
une troisième couche adhésive (42) fournie sur le second substrat sur un côté du corps vivant,
dans lequel la troisième couche adhésive et la première couche adhésive forment une surface de fixation à fixer sur le corps vivant.

7. Capteur biologique selon la revendication 1 ou la revendication 6,
dans lequel l'électrode possède un trou traversant (31) à travers lequel la première couche adhésive peut être exposée dans un état dans lequel l'électrode est fixée à la première couche adhésive.
